# EUROPEAN PATENT APPLICATION

(11) **EP 4 837 547 A1**
(43) Date of publication of application: **11.11.2026**
(21) Application number: 25382471.8
(22) Date of filing: 09.05.2025
(51) Int. Cl.: A61F 13/0246, A61L 15/26, A61L 15/58

(54) **ATRAUMATIC AND EASY-TO-USE MEDICAL TAPE WITH A POLYURETHANE RELEASE FILM TO FACILITATE UNWINDING FROM A SPOOL**

(71) Applicant: Paul Hartmann AG, 89522 Heidenheim (DE)
(72) Inventor: Guillermin, Mélanie, 38230 Charvieu (FR); Lecoeuvre, Jean-François, 42290 Sorbiers (FR); Gonzáles Gómez-Arevalillo, Fernando, 08030 Barcelona (ES); Rovira, Josep, 08223 Terrassa (ES)
(74) Representative: Paul Hartmann AG Patents & Licensing

(57) **Abstract**

The present invention relates to a medical tape (1) for fixation of a wound dressing. The medical tape (1) comprises a nonwoven structural layer (2) with a top side (3) and a bottom side (4). A release film (5) comprising a polyurethane is attached to the top side (3) of the structural layer (2) and an adhesive silicone gel (6) is coated onto the bottom side (4) of the structural layer (2). In addition, the present invention relates to a spool (9) with the medical tape (1) and a method of manufacturing the medical tape (1). Thus, the present invention provides a comfortable medical tape suitable for sensitive skin, which is characterized in particular by its atraumatic adhesive properties and its ease of use.

## Description

The present invention relates to a medical tape for fixation of a wound dressing. In addition, the present invention relates to a spool with the medical tape and a method of manufacturing the medical tape.

Adhesive medical tapes or plasters are known in the art. They can advantageously be provided wound up on a spool and used, for example, to attach a wound dressing to a wound site. A disadvantage of some adhesive tapes currently available on the market may be that they are equipped with a strong adhesive. This can make it painful for the patient when the tape is removed from the wound site, for example when changing the dressing. In addition, some adhesive tapes cannot be torn by hand, so that sections of the tape have to be cut with scissors. However, this makes the adhesive tape more difficult to use.

Accordingly, the object of the present invention was to provide an improved medical tape which does not have the aforementioned disadvantages, or at least to a lesser extent. In particular, the object of the present invention was to provide an improved medical adhesive tape that can be removed from a patient's body with minimal pain, i.e. that is designed to be atraumatic, while also being easy to use. The above-mentioned problems are solved by a medical adhesive tape according to claim 1 and a manufacturing method according to claim 13.

The medical tape according to the present invention is intended for fixation of a wound dressing at a wound site. The medical tape comprises a nonwoven structural layer with a top side and a bottom side. A release film comprising a polyurethane is attached to the top side of the structural layer. The release film is a plastic film comprising or consisting of polyurethane, which is non-detachably and directly bonded to the top side of the structural layer. The designation of this plastic film as a "release film" is related to its function, which will be described in more detail below. Moreover, an adhesive silicone gel is coated onto the bottom side of the structural layer. In other words, the bottom side of the structural layer is coated with an adhesive silicone gel. Thus, the present medical tape comprises a nonwoven structural layer having a top side and a bottom side, the top side being bonded to a release layer formed by a plastic film comprising a polyurethane and the bottom side being bonded to an adhesive layer formed by an adhesive silicone gel.

By using a nonwoven as the structural layer, the present medical tape can advantageously be soft, breathable and tearable by hand. Furthermore, the adhesive silicone gel imparts atraumatic properties to the medical tape, as well as the ability to be repositioned. At the same time, the adhesive force of the medical tape on intact skin is still sufficient to permanently and securely attach a wound dressing to the wound site. Attaching the release film to the top side of the structural layer of the medical tape has the effect that several layers of the medical tape can be placed on top of each other and releasably bonded to each other. This is what makes it possible to provide the present medical tape wound on a spool and to easily unwind it from the spool. A separate protective layer removably attached to the adhesive silicone gel is not required in the present invention. The present medical tape can also be advantageously water-repellent due to the outer release plastic film. Overall, the present invention thus provides a comfortable medical tape suitable for sensitive skin, which is characterized in particular by its atraumatic adhesive properties and its ease of use. Further advantages of the invention are explained in the following preferred embodiments.

Typically, the medical tape consists of the nonwoven structural layer, the release film and the adhesive silicone gel. Therefore, the medical tape can be simple and consist of only a few components. This embodiment does not rule out the possibility of the medical tape being wound onto a spool as proposed below.

In a preferred embodiment of the present invention, the medical tape has a peel adhesion of 2.2 to 4.0 N/25 mm. More preferably, the medical tape has a peel adhesion of 2.2 to 3.5 N/25 mm. In particular, the medical tape has a peel adhesion of approximately 3.0 N/25 mm or 3.2 N/25 mm. The peel adhesion may be determined according to FINAT No. 1. In particular, the peel adhesion may relate to the peel adhesion at 180° on Bristol paper, determined according to FINAT No. 1. The Bristol paper may be purchased from the company Exacompta, article number 13316E. With such a peel adhesion, the medical tape can securely fix wound dressings while also being atraumatic.

Surprisingly, it has been shown that the medical tape can have a comparatively high water vapor permeability (moisture vapor transmission rate, MVTR) and thus breathability. In general, high breathability is advantageous to avoid maceration of the skin at the wound site due to fluid accumulation. For instance, the medical tape may have a moisture vapor transmission rate of 460 to 530 g/m²/24h. In particular, the medical tape may have a moisture vapor transmission rate of approximately 495 g/m²/24h. The moisture vapor transmission rate may be determined according to DIN EN 13726-2:2002-06 (chapter 3.2).

The medical tape may have a release force of 0.5 to 1.2 N/25 mm. In particular, the medical tape may have a release force of approximately 0.8 N/25 mm. The release force may be determined according to the test method described in the present specification. The release force may indicate the force needed to separate an adhesive tape from the back side of a similar tape. With a release force as previously indicated, the adhesive tape can easily be unwound from a spool.

Moreover, the medical tape may have a tear resistance of 1.3 to 1.8 N/25 mm. In particular, the medical tape may have a tear resistance of approximately 1.5 N/25 mm. The tear resistance may be determined according to DIN EN ISO 9073-4:1997-09. With such tear resistance, the medical tape can be torn by hand and thus be easy to use.

As already mentioned, the present medical tape comprises a nonwoven structural layer. In the following, preferred features of the structural layer are provided, which, for example, can be advantageous for achieving the desired breathability and tearability of the medical tape. Preferably, the nonwoven structural layer comprises cellulose fibers and polyester fibers. In particular, the polyester fibers may consist of polyethylene terephthalate (PET). Furthermore, the nonwoven structural layer may be a wet-laid, chemically bonded nonwoven. The nonwoven structural layer may also comprise a styrene-acrylic copolymer as a binder.

The nonwoven structural layer can be further specified in terms of its thickness. Accordingly, the nonwoven structural layer may have a thickness of 90 to 120 µm. In particular, the nonwoven structural layer may have a thickness of approximately 105 µm. The thickness may be determined according to NF EN ISO 534: 2005.

The nonwoven structural layer can also be further specified in terms of its grammage. For example, the nonwoven structural layer may have a grammage of 35 to 45 g/m². In particular, the nonwoven structural layer may have a grammage of approximately 40 g/m². The grammage may be determined according to ISO 3801.

The nonwoven structural layer can also be further specified in terms of its air permeability. For instance, the nonwoven structural layer may have an air permeability (at 196 Pa) of 280 to 840 l/m²/s. In particular, the nonwoven structural layer may have an air permeability of approximately 560 l/m²/s. The air permeability may be determined according to WSP 70.1 (05).

A nonwoven suitable for the structural layer of the present invention can be obtained from the company Ahlstrom Munksjö (Brignoud, France) under the designation BR7378040.

Another essential component of the medical adhesive tape according to the invention is the aforementioned release film, which is described in more detail in the following embodiments of the invention. Typically, the release film is anti-adhesive. This means that the release film typically does not contain any adhesive components. That is why it is also referred to as a "release film". Usually, the release film completely covers the top side of the structural layer. Moreover, the release film is normally transparent. In a particularly preferred embodiment of the present invention the release film is attached to the top side of the structural layer by means of hot lamination. In addition, the release film may have a thickness of 20 to 60 µm.

In terms of composition, the release film can essentially consist of the polyurethane. The release film can then also be referred to as a polyurethane release film. Preferably, the polyurethane of the release film is a hydrophilic ester resin. Typically, the release film or the polyurethane of the release film does not contain any silicone component. Accordingly, the polyurethane used in the present invention is typically not a silicone polyurethane. This can save costs. Similarly, the release film or the polyurethane of the release film typically does not contain any fluorine component.

A release film suitable for the present invention can be purchased, for example, from Covestro (Leverkusen, Germany) or Faitplast (Cellatica, Italy).

The adhesive silicone gel of the medical tape is described in more detail below. Normally, the adhesive silicone gel forms a continuous layer on the bottom side of the structural layer. This layer of the adhesive silicone gel may have a thickness of 80 to 120 µm. Moreover, the adhesive silicone gel may have a coating weight of 80 to 120 g/m². The adhesive silicone gel may advantageously have a gel hardness of 100 to 220 mm/10, for example of approximately 155 mm/10 or approximately 185 mm/10, as measured according to DIN ISO 2137 (hollow cone 62.5 g).

Regarding its chemical composition, the adhesive silicone gel of the present invention is a crosslinked silicone polymer with a gel-like consistency. The silicone gel is typically formed using a hydrosilation (or polyaddition) reaction between an alpha-omega vinyl terminated polydimethyl siloxane and a Si-H containing siloxane catalyzed by a platinum catalyst. Thermal curing may preferably be applied to accelerate the polymerization process. In addition, the silicone gel may be solvent free.

Adhesive silicone gels are known in the prior art, for example from US 2007/0270555 A1, and are commercially available. Generally, silicone systems to produce such silicone gels are sold by silicone manufacturers as two-component kits. The two components of the kit are mixed with each other to form the gel and may comprise:

| **Part A** | **Part B** |
|---|---|
| - Mix of alpha-omega vinyl terminated polydimethyl siloxane oils | - Mix of alpha-omega vinyl terminated polydimethyl siloxane oils |
| - Pt catalyst | - Mix of Si-H containing siloxane catalyzed oils |
| | - Pt catalyst inhibitor (i.e. ethynyl cyclohexanol and the like) |

The platinum catalyst inhibitor stabilizes the final mix, avoiding too early crosslinking of the product during coating. This volatile chemical will evaporate during curing at high temperature (100 to 180°C). Silicone systems suitable for the present invention may be purchased from Elkem Silicones (Silbione RT Gel range or Silbione HC2 range), Wacker Silicones (Silpuran range), Dupont Liveo (soft skin adhesives MG7 range), Momentive (Silopren Gel range) and Nusil (Med Gels range). Particularly preferred is the Silbione RT Gel range from Elkem Silicones (for example Silbione RT Gel 4640 A&B) and the Silpuran range from Wacker (for example Silpuran 2114 A/B).

The present medical tape is particularly intended to be provided wound on a spool. Accordingly, a spool with the medical tape according to the invention is also claimed. In other words, a medical article comprising or consisting of a spool and the medical tape according to the invention is also claimed, wherein the medical tape is wound on the spool. When the medical tape is wound onto the spool, it forms a roll. In this way, a long strip of medical tape can be provided in an easily manageable form. As required, the user can then quickly and easily tear off different-sized pieces of tape from the roll by hand. Alternatively, the user can also cut the tape to size with scissors if they prefer.

The spool may be formed so that a medical tape with the dimensions (width x length) of 1.25 cm x 5 m, 2.5 cm x 5 m, 5 cm x 5 m, 1.25 cm x 9.2 m, 2.5 cm x 9.2 m or 5 cm x 9.2 m can be wound onto it.

The spool may be, in particular, a disc spool. A disc spool as defined herein comprises a substantially cylindrical core ("core tube") having disc-like, protruding boundaries attached to its end faces. The medical tape is then wound onto the cylindrical core. The roll of tape that is formed is limited, protected and supported by the discs of the spool. Such spools are widely used for such purposes and are known to those skilled in the art.

As already mentioned, the present medical tape is used in particular to attach wound dressings to a patient's body. Therefore, the present invention also relates to the use of the present medical tape (or spool with the tape) to fix a wound dressing on a body of a patient. Due to its particularly advantageous atraumatic properties, it is also possible to apply the adhesive tape to sensitive skin. The wound dressing may be any wound dressing material which requires additional adhesive fixation and is suited to be used in combination with the present medical tape. For instance, the wound dressing may be a compress. Furthermore, the present medical tape can also be used to attach other medical objects such as a cannula, a catheter or a tube to a patient's body.

Another subject of the present invention relates to the manufacture of the medical tape. According to this subject, a method of manufacturing the medical tape (or spool with the tape) is provided, which comprises the following steps:
i. providing a nonwoven structural layer with a top side and a bottom side,
ii. providing a release film comprising a polyurethane,
iii. attaching the release film to the top side of the structural layer,
iv. coating of the bottom side of the structural layer with a composition forming an adhesive silicone gel layer to obtain the medical tape,
v. optionally, providing a spool, and
vi. optionally, winding of the medical tape on the spool.

With regard to method steps iii. and iv. it is recommended that the steps be carried out in the given order to achieve optimal results. Thus, the top side should first be bonded to the release film as indicated and only then should the bottom side be coated with the silicone composition.

The silicone composition used in method step iv. may comprise an alpha-omega vinyl terminated polydimethyl siloxane, a Si-H containing siloxane and a platinum catalyst.

As already mentioned, the release film is preferably attached to the structural layer by means of hot lamination. The silicone composition may be directly coated onto the structural layer. However, it is more advantageous if the silicone composition is applied to the structural layer by means of transfer coating, in particular transfer coating in combination with a corona treatment. Transfer coating is known in the prior art and described, for example, in US 9,827,206 B2. The transfer coating may prevent or at least reduce an undesired penetration of the coating material into a porous substrate like the present nonwoven structural layer. With regard to the present silicone gel layer, the transfer coating may allow a corona treatment of the silicone gel. This advantageously may improve the adhesion of the silicone gel on the nonwoven structural layer.

If in step iv. the transfer coating is carried out, the silicone composition is not directly applied to the bottom side of the structural layer. Instead, the composition is first applied to a separate substrate layer (for example an anti-adherent film), from which it is afterwards transferred to the structural layer. The transfer may be carried out after complete formation of the crosslinked silicone gel and preferably a corona treatment as disclosed in US 9,827,206 B2.

According to a preferred embodiment of the manufacturing method, hot air or infrared radiation is applied to the silicone composition to accelerate the formation of the adhesive silicone gel layer. In this way, the manufacturing speed of the medical tape can be increased advantageously.

The aforementioned spool with the medical tape, use of the medical tape and manufacturing method of the medical tape also refer to the specific embodiments previously (only) described in connection with the medical tape. That is, the further features of the previously described specific embodiments of the medical tape are also part of the aforementioned spool, use and manufacturing method.

### Figures to illustrate the invention

The figures shown are intended to illustrate the present invention by way of example. The figures merely represent embodiments of the invention and should therefore in no way be understood as limiting it. The same structural components may have the same reference signs in the figures.

### Brief description of the figures

**Figure 1** shows an embodiment of a medical adhesive tape according to the invention in a side-cut view.
**Figure 2** shows how the tape of figure 1 overlaps itself when it is wound onto a spool.
**Figure 3** shows a preferred embodiment of a spool on which the tape of figure 1 is wound.

### List of reference signs

- **1**: medical tape
- **2**: structural layer
- **3**: top side of the structural layer
- **4**: bottom side of the structural layer
- **5**: release film / release layer
- **6**: adhesive silicone gel (layer)
- **7**: a section of a first winding of the medical tape provided on a spool
- **8**: a section of a second winding of the medical tape provided on a spool
- **9**: disc spool

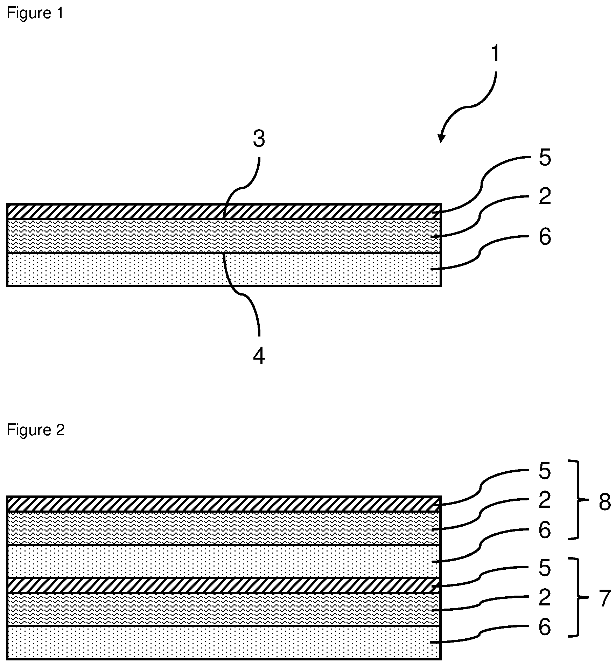

**Figure 1** shows an embodiment of a medical adhesive tape **1** according to the invention in a side-cut view. The tape **1** comprises a structural layer **2** formed from a nonwoven having a top side **3** and a bottom side **4.** A release film **5** comprising a polyurethane is attached to the top side **3.** The bottom side **4** is coated with an adhesive silicone gel **6.** The release film **5** and the silicone coating **6** are formed in such a way that they form continuous layers on the top and bottom sides **3, 4** of the structural layer **2.** In use, the tape **1** is attached to the patient's body by means of the adhesive silicone gel **6.** In use, the release film **5** is arranged facing away from the patient's body and forms an outer side of the tape **1.**

The tape **1** is usually provided rolled up on a spool. In this case, a strip of the tape **1** is attached to the spool with the adhesive silicone gel 6 and then wound onto itself. This results in a roll of tape **1** in which the tape **1** comes to rest on itself and thus overlaps itself. This overlapping of the tape **1** is schematically shown in **Figure 2** by means of a first winding **7** and a second winding **8** overlapping the first winding **7.** As shown in Figure 2, the adhesive silicone gel **6** of the second winding **8** is attached to the release film **5** of the first winding **7.** However, due to the anti-adhesive nature of the release film **5,** the tape **1** can be easily detached from the underlying first winding **7** in the area of the second winding **8.** This makes it easy to unwind the tape **1** from the spool.

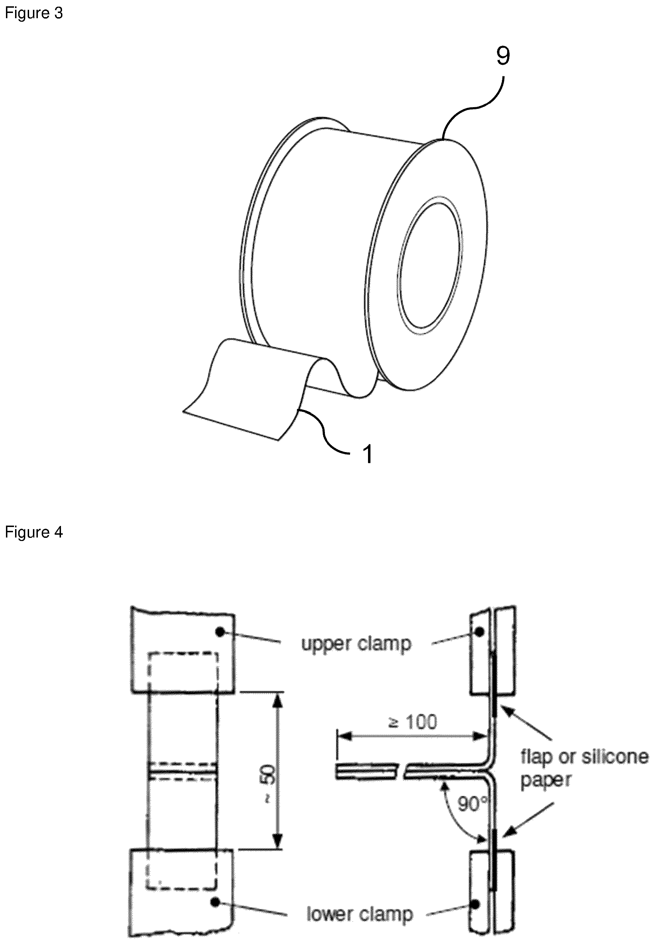

Finally, **Figure 3** shows a preferred embodiment of a spool **9** on which the tape **1** is wound as described above. The spool **9** is a disc spool known per se from the prior art. The spool **9** can, for example, be dimensioned such that it can hold a strip of the tape **1** with a dimension of 1.25 cm (width) x 5 m (length).

### Determination of the release force

In the following, a method how the release force may be determined is described.

### Scope

Testing of adhesive plasters / tapes. The release force is the force needed to separate an adhesive tape from the back side of a similar tape under specified conditions.

### Testing device/ tools

- tensile testing machine according to DIN EN ISO 7500-01:2016-05, class 1
- scissors
- tape applicator (Sondes Place Research Institute; Dorking Surrey England; Type No. D 427/1), prepared for a roll over with 20 N/cm tape width
- punching knife: width 25 mm ± 0.25 mm
- silicone paper
- stopwatch
- glass or steel plates

### Sample preparation

1. before testing put samples (in full length and width in tensionless state) in the specific standard atmosphere (23 ± 2°C, 50 ± 4 % rH) for at least 24 hours in such a way that the air has free access
2. glue unrolled plaster strips onto the silicone paper
3. punch strips of 25 mm width and approx. 200 mm length
   Notes:
   - the lower strip of the laminate can be wider than the upper strip
   - two plaster strips are required for each test

### Test procedure

1. in general, perform five measurements (n = 5)
2. test with the following parameters:

| pre-load [N] | testing speed [mm/min] |
|---|---|
| 0.1 | 300 |

3. apply light finger pressure to glue one of the punched-out strips to the carrier material of the second strip
4. on one side of the plaster strips the ends are peeled off and glued to each other; two flaps are produced
5. place the test specimen on a glass or steel plate and roll 2 x in both longitudinal directions with the tape applicator; mark the beginning of the rolling path near the flaps by a line on the carrier material
6. observe 10 minutes of rest time
7. carefully peel off flaps to the mark
8. fix a flap into the upper and lower clamp each so that the two test strips are separated at an angle of 90° over a measuring distance of approximately 180 mm (see **Figure 4****)**

### Evaluation

- release force as arithmetic mean of the force peaks in cN/cm (or N/25 mm), rounded to one decimal place
- optionally, median of the force peaks in cN/cm, rounded to one decimal place
- optionally, range between maximum and minimum force peak in cN/cm, rounded to one decimal place

### Reference standards

- tensile testing machine according to DIN EN ISO 7500-01:2016-05, class 1
- specific standard atmosphere according to DIN EN ISO 139:2011-10
- evaluation of the peel test based on DIN ISO 6133:2017-04, procedure B

## Claims

1. Medical tape (1) for fixation of a wound dressing comprising a nonwoven structural layer (2) with a top side (3) and a bottom side (4),
wherein a release film (5) comprising a polyurethane is attached to the top side (3) of the structural layer (2) and
wherein an adhesive silicone gel (6) is coated onto the bottom side (4) of the structural layer (2).

2. Medical tape (1) according to claim 1, wherein the tape (1) consists of the nonwoven structural layer (2), the release film (5) and the adhesive silicone gel (6).

3. Medical tape (1) according to claim 1 or 2, wherein the tape (1) has a peel adhesion of 2.2 to 4.0 N/25 mm, preferably of 2.2 to 3.5 N/25 mm.

4. Medical tape (1) according to anyone of the preceding claims, wherein the tape (1) has a moisture vapor transmission rate of 460 to 530 g/m²/24h.

5. Medical tape (1) according to anyone of the preceding claims, wherein the tape (1) has a release force of 0.5 to 1.2 N/25 mm.

6. Medical tape (1) according to anyone of the preceding claims, wherein the tape (1) has a tear resistance of 1.3 to 1.8 N/25 mm.

7. Medical tape (1) according to anyone of the preceding claims, wherein the release film (5) is attached to the top side (3) of the structural layer (2) by means of hot lamination.

8. Medical tape (1) according to anyone of the preceding claims, wherein the release film (5) essentially consists of the polyurethane.

9. Medical tape (1) according to anyone of the preceding claims, wherein the polyurethane of the release film (5) is a hydrophilic ester resin.

10. Medical tape (1) according to anyone of the preceding claims, wherein the adhesive silicone gel (6) forms a continuous layer on the bottom side (4) of the structural layer (2), wherein the layer of the adhesive silicone gel (6) preferably has a thickness of 80 to 120 µm.

11. Spool (9) with the medical tape (1) according to anyone of the preceding claims.

12. Use of the medical tape (1) according to anyone of the claims 1 to 10 to fix a wound dressing on a body of a patient.

13. Method of manufacturing the medical tape (1) according to anyone of the claims 1 to 10, comprising the steps of:
i. providing a nonwoven structural layer (2) with a top side (3) and a bottom side (4),
ii. providing a release film (5) comprising a polyurethane,
iii. attaching the release film (5) to the top side (3) of the structural layer (2),
iv. coating of the bottom side (4) of the structural layer (2) with a composition forming an adhesive silicone gel layer (6) to obtain the medical tape (1),
v. optionally, providing a spool (9), and
vi. optionally, winding of the medical tape (1) on the spool (9).

14. Method according to claim 13, wherein the release film (5) is attached to the structural layer (2) by means of hot lamination.

15. Method according to claim 13 or 14, wherein the composition is applied to the structural layer (2) by means of transfer coating, in particular transfer coating in combination with a corona treatment.
